(19) Europäisches Patentamt
European Patent Office
Office européen des brevets

(11) EP 0 882 002 B1

(12) EUROPEAN PATENT SPECIFICATION

(45) Date of publication and mention
of the grant of the patent:
**06.11.2002 Bulletin 2002/45**

(51) Int Cl.[7]: **C07C 5/31**

(86) International application number:
**PCT/US96/14284**

(21) Application number: **96932176.9**

(22) Date of filing: **05.09.1996**

(87) International publication number:
**WO 97/009290 (13.03.1997 Gazette 1997/12)**

(54) **PROCESS FOR SELECTIVELY OPENING NAPHTHENIC RINGS**

VERFAHREN ZUR SELEKTIVEN SPALTUNG VON NAPHTHALIN-RINGEN

PROCEDE D'OUVERTURE SELECTIVE D'ANNEAUX NAPHTHENIQUES

(84) Designated Contracting States:
**BE DE FR GB IT NL**

(30) Priority: **05.09.1995 US 523300**

(43) Date of publication of application:
**09.12.1998 Bulletin 1998/50**

(73) Proprietor: **ExxonMobil Research and
Engineering Company
Annandale, New Jersey 08801 (US)**

(72) Inventors:
• **TOUVELLE, Michele, S.
Baton Rouge, LA 70808 (US)**
• **McVICKER, Gary, B.
Califon, NJ 07830 (US)**
• **DAAGE, Michel
Baton Rouge, LA 70810 (US)**
• **HANTZER, Sylvain, S.
Prairieville, LA 70769 (US)**
• **HUDSON, Carl, W.
Baton Rouge, LA 70815 (US)**
• **KLEIN, Darryl, P.
Baton Rouge, LA 70820 (US)**

• **VAUGHAN, David, E., W.
Flemington, NJ 08822 (US)**
• **ELLIS, Edward, S.
Basking Ridge, NJ 07920 (US)**
• **CHEN, Jingguang, G.
Somerville, NJ 08876 (US)**

(74) Representative: **Dew, Melvyn John et al
ExxonMobil Chemical Europe Inc.
Law Technology
P.O.Box 105
1830 Machelen (BE)**

(56) References cited:
**US-A- 3 617 511        US-A- 4 676 885
US-A- 4 783 575        US-A- 4 834 866
US-A- 5 334 792        US-A- 5 345 026
US-A- 5 463 155**

Remarks:
The file contains technical information submitted
after the application was filed and not included in this
specification

**Description**

**Field of the Invention**

The present invention relates to a process for selectively opening organic ring compounds in a feedstream wherein at least a portion of the ring compounds in the feedstream are characterized as containing at least one $C_5$ ring having at least one substituent therefrom and wherein the number of carbon atoms for the $C_5$ ring compound is at least 8. The $C_5$ naphthenic rings are opened without significant dealkylation of any pendant substituents on the ring.

**Background of the Invention**

There is an increasing demand for environmentally friendly hydrocarbons and clean-burning high performance fuels, such as distillate fuels like diesel and jet fuels. Distillate fuels typically contain paraffins, naphthenes, and aromatics. For fuel quality parameters such as cetane, gravity and emissions, paraffins are the most desirable components, followed by naphthenes, followed by aromatics. The least desirable are multi-ring aromatic compounds. While various refinery processes produce distillate fuels, these processes are typically limited in their capability to produce high quality distillate fuel and/or high yields of distillate fuel. For example, conventional hydrogenation processes saturate aromatic rings to naphthenes, thereby increasing the cetane number and increasing the API gravity (lower density). The disadvantage of hydrogenation alone is that naphthenes have generally lower cetane values and are more dense than paraffins having substantially the same number of carbon atoms. The greater density of naphthenes results in reduced volume of the distillate fuel blend relative to a composition containing similar concentrations of paraffins instead of naphthenes. Similarly, multi-ring naphthenes are generally more dense and have lower cetane values than single-ring naphthenes having substantially the same number of carbon atoms. In addition, naphthenes can be converted to aromatics via oxidation reactions. Since combustion of naphthenes in fuels occurs under oxidizing conditions, there is the potential for naphthenes to revert to aromatics under combustion conditions, thus further reducing fuel quality.

Another conventional refinery process for producing distillate fuels is hydrocracking. Hydrocracking catalysts are typically composed of hydrogenation metals supported on acidic supports, such as zeolites. These catalysts are effective, under typical process conditions, for extensive hydrogenation of aromatics and for reducing the number of ring structures, however with the accompanying liability of extensive cracking to lower boiling products including gases which lowers the overall boiling range and limits the volume of final distillate product. In fact, analyses of distillate boiling range paraffin content in hydrocracking feeds versus products suggest little net increase in production of these paraffins via hydrocracking, rather concentration of paraffins in the final product through the cracking of ring structures to lower molecular weight compounds which no longer reside in the distillate boiling range. Thus, the apparent increase in distillate boiling range paraffins and improved distillate fuel quality can result primarily from a combination of hydrogenation of aromatics and a concentration of paraffins in a reduced volume of distillate product.

There is also an increasing demand for low toxicity, biodegradable solvents, of which paraffins are a preferred class. Consequently, it is desirable to reduce the cyclic compound content of hydrocarbon solvent blends, in general. and to convert naphthenes to paraffins, in particular. While there are descriptions of ring opening reactions in the prior art, owing to the increasing demand for more environmentally friendly solvents and clean-burning, high performance fuels, it is highly desirable to have a process which is more selective for ring opening than currently available. Selectivity for ring opening is related to the propensity for cleavage of a ring bond which results in product molecules having an equivalent number of carbon atoms and one less ring than the original molecule, rather than cleavage of a bond which results in a product molecule having fewer carbons than the original molecule. A perfectly selective ring opening process would give only ring bond cleavage to produce molecules having an equivalent number of carbon atoms and one less ring than the original molecule. For example, from a hydrocarbon stream containing only single ring naphthenes of n number of carbon atoms, the product from perfect ring opening selectivity would be only paraffins of n number of carbon atoms.

Thus, the greater number of product molecules from a ring opening process having an equivalent number of carbon atoms and one less ring than the original molecule, the greater the selectivity for ring opening. Greater selectivity for ring opening is important for the reasons that a) the number of ring structures in a product stream will be decreased, b) significant dealkylation of any pendant substituents on the ring, which will reduce the volume of product in a specified boiling range, will be minimized and c) the density of the product stream will be decreased providing volume swell. The present invention provides for a ring opening process, and the criteria for selecting a catalyst for that process, with significantly higher selectivity for ring opening than those practiced in the current art. with the object to decrease the number of ring structures in a product stream and decrease the density of the product stream to improve volume swell, while minimizing dealkylation of pendant substituents to maintain a high volume of product in the desired boiling range.

There are a number of references to ring opening in the the prior art. Most references fall into two general categories - hydrogenolysis and hydrogenation/hydrocracking. "Hydrogenolysis", for purposes of this invention, is defined as cleavage of a carbon-carbon bond, with addition of hydrogen at each point of cleavage. Most references in this category deal with reactions of small, single ring naphthenes over primarily noble metal catalysts.

"Hydrogenation/hydrocracking" is generally practiced on larger cyclic molecules over primarily acidic zeolite-supported noble and other Group VIII metal catalysts. The more selective ring opening process of the present invention differs in that the catalyst is selected from those which give higher hydrogenolysis activity and selectivity for ring opening than previously recognized or anticipated. Furthermore, they provide significantly less substituent and secondary product cracking in ring opening than observed in conventional hydrocracking processes.

Much academic research has sought to elucidate key mechanisms controlling product distributions in hydrogenolysis and hydrocracking reactions by using model compounds and specific noble metal catalyst types. Typical studies have been reviewed by Gault (Adv. Catal., 30, 1-95, (1981) with a particular attention to $C_6$ ring isomers, including a description of skeletal isomerization of hydrocarbons over metal catalysts and a discussion of the mechanisms of ring opening reactions. The greater ease for ring opening $C_5$ versus $C_6$ cycloparaffin rings and mechanistic implications related to which bond in the $C_5$ ring is cleaved are described.

The isomerization of $C_6$ ring (cyclohexane) to $C_5$ ring (methylcyclopentane) was demonstrated as a step preceding ring opening (the pentyl-ring opening much faster than the hexyl-ring) by Schultz and co-workers (Proc. 5th Intl. Catal. Congr., North-Holland Publ. (Aidam), v.2, 1229-39, (1973)). The tendency for side chains on ring structures to fragment and to isomerize methyl groups to other ring sites (the so called "paring" reaction) has also been demonstrated (Egan. et al. J. Amer. Chem. Soc., 84, 1204-12. (1962). The latter process, which results in tertiary carbon atoms on the ring, severely inhibits ring opening at those sites and is in agreement with the findings of Gault. These processes are characteristic of those using a wide range of bifunctional metal hydrogenation-acidic catalysts. For instance, various Pt-cation exchanged acidic zeolites have been demonstrated to be effective for naphthene isomerizations using cycloparaffins with short side chains (Weitkamp, et al. in "Structure and Reactivity of Modified Zeolites", Elsevier (Adam), 279-90, (1984)). Such naphthene isomerizations are also well demonstrated on non-noble metal, non-zeolite catalysts (NiS on amorphous silica-alumina) for longer side-chain $C_9$ to $C_{12}$ alkyl-napthenes (Egan, et al, J. Amer. Chem. Soc.. 84, 1204-12 (1962)). To meet distillate quality targets, control of the paring isomerizations and subsequent dealkylations are particularly important in order to limit the number of lower cetane, highly branched paraffins which may result following ring opening.

Other references to ring opening include U.S. Pat. No. 3,617,511 which teaches a catalyst comprised of rhodium or ruthenium on an acidic refractory oxide, specifically a halogen-promoted alumina, for ring opening of cycloparaffins. Greater selectivity for ring opening methylcyclopentane (MCP) versus cyclohexane (CHx) in admixture was observed. In addition, essentially sulfur free feeds were preferred.

Further, U.S. Pat. Nos. 4,783,575 and 4,834,866 disclose the use of a chlorided platinum-alumina catalyst to isomerize $C_4$-$C_6$ paraffins to more highly branched isomers and to ring open cycloparaffins found in the feedstock. Continuous addition of chloride to maintain catalyst acidity and low severity conditions to minimize secondary cracking were preferred. Platinum was found to be most suitable of the catalytic metals.

Also, U.S. Pat. No. 3,631,117 describes a process for the hydroisomerization of cyclic hydrocarbons, specifically isomerization of $C_6$ ring cycloparaffins to $C_5$ ring isomers, employing a zeolite-supported Group VIII metal catalyst for hydroisomerization of cyclics. A broad range of Group VIII metals alone or in combination with each other or with tungsten are claimed. Notably, iridium was absent from this group. The conditions for this hydroisomerization process also provided some ring opening and paraffin isomerization. A note of caution was made that excessive hydrocracking (reducing the number of carbon atoms from the original cyclic molecule) can be a problem under the conditions of $C_6$, ring to $C_5$ ring hydroisomerization.

The sensitivity for cracking of a n-butyl side chain from a $C_5$ ring with platinum on carbon catalysts has been noted (Sergienko, et al., Khim. Geol, Nauk 2, 65-70 (1976)). At relatively mild conditons ($225°C$ to $270°C$) low yields of $C_9$ paraffins were produced with good selectivity. However, as conversion increased, through either higher platinum loadings or higher temperature, significant amounts of hydrocracking and aromatic products were formed.

There are also patents which teach ring opening in naphtha feeds. For example. U.S. Pat. No. 5,334,792 discloses a two stage process wherein a naphtha feedstock is reacted in a first stage with a zeolite catalyst containing a hydrogenation component under conditions which will saturate aromatics. such as benzene, and open cyclic hydrocarbons. The reaction product from the first stage is passed to a second stage containing an isomerization catalyst to isomerize paraffins to obtain higher octane products. Also, process of U.S. Pat. No. 5,345,026 comprises contacting cyclic hydrocarbons with a catalyst under sufficient ring opening conditions wherein the catalyst is comprised of: (i) a hydrogenation/dehydrogenation component; and (ii) an acid component comprised of a Group IVB metal oxide modified with an oxyanion of a Group VIB metal.

While hydrocracking can reduce the number of ring compounds in the final distillate product with an attendant increase in the cetane number, the yield of product boiling in the distillate range is significantly reduced by excessive cracking to lower boiling products, including gases. One reason for excessive cracking is that paraffins and paraffinic side chains derived from previously ring opened naphthenes, crack more readily than the starting and remaining napthenes. Comparative analyses of distillate boiling range paraffin contents in both hydrocracking feeds and the resulting products suggests little net increase in paraffins, but rather a concentration of such paraffins in the final product because products from secondary cracking of the opened naphthene rings end up in a lower boiling fraction, outside of the distillate range. Thus, the apparent increase in distillate paraffins, and thus improved distillate fuel quality results primarily from a combination of aromatics saturation and a concentration of paraffins in a reduced volume of product within a given boiling range.

Most recent developments in hydrocracking catalysts have focused on matching the hydrogenation of catalytic noble metals with an acid cracking function. The acid function was provided in earlier catalysts by an amorphous alumina or silica-alumina, and more recently by a crystalline zeolite. The metal function is typically provided by Pt and/ or Pd, although all noble metals are treated as functionally equivalent in the patent art. The zeolite component is typically a modified Y-type (US Patent 3,130,007), usually derived from a steamed variety designated "ultrastable-Y", or simply US-Y (US Patent 3,449,070). The art comprises numerous combinations of these two "matched" components, most of which were recently reviewed by Ward (Fuel Process. Technol., 35, 55-85(1993)) who described in detail the manipulation of product slates by changing catalysts. Key differentiating characteristics are in the variable combinations of Pt and Pd (one or both metals, relative loading, dispersion, distribution between zeolite and matrix) and the particular way in which the US-Y has been processed. The latter components are usually defined by low unit cell values, high Si/Al ratios, residual exchange cation contents and sometimes pore volumes. This last property is determined by the methods and intensity for dealuminating which determines the distribution of mesopores within the remnant zeolite crystal and the retained crystalline micropore volume. Ward (ibid) has authoritatively reviewed the many differences in selectivities between the zeolite products of these numerous process variations.

Attempts have been made to increase hydrocracking selectivity via hydrodecyclization. For example. European Patent Application EP0512652 A1 describes a "hydrodecyclization" process wherein the distillate fuel is contacted at elevated temperatures with a suitable catalyst in the presence of hydrogen, which catalyst is comprised of one or more Group VIII noble metals on a modified Y-type zeolite support having a unit cell size between 2.42 and 2.44 nm (24.20 Å and 24.40 Å) and a $SiO_2/Al_2O_3$ molar ratio of 10 to 150. Similarly, European Patent Application EP0519573 A1 teaches a process for reducing cyclic structures similar to the above EP application except that an alkali or alkaline-earth metal is also present. The objective was to improve the cetane number of distillate fuels by opening rings (hydrodecyclization) without excessive cracking. While these two European patent applications suggest that ring opening is taking place, there is no direct evidence in said applications to show that selective ring opening is the reason for improved distillate product quality. Based on process conditions and product yields and qualities provided in the examples, it is more likely that the reported increase in distillate boiling range paraffins and improved distillate fuel quality results primarily from a combination of extensive hydrogenation of aromatics and a concentration of paraffins in a reduced volume of product.

This is in agreement with the observations of Mignard, et al., who studied the opening of naphthenic molecules over a platinum on Y zeolite catalyst under hydrocracking conditions ("Catalytic and Hydroprocessing of Petroleum and Distillates," M. Decker (New York), 447-459 (1994)). The reaction pathway for ring opening of cycloparaffins was described as sequential isomerization of $C_6$ to $C_5$ ring, followed by carbon-carbon bond cleavage to give ring opening, followed by rapid cracking via additional carbon-carbon bond cleavage reactions. The results showed cracking propensity increases with increasing carbon number and that ring opened products are highly susceptible to further cracking. The conclusion reached was that ring opening selectivity to minimize cracking is difficult to control. Indeed these authors suggest that the present state of the art is representative of the limits inherent in the competing reactions involved in hydrocracking under the allowable process conditions.

Hydrocracking catalysts are bifunctional in nature, containing both metal and acidic functionalities. Balancing the relative activity of these functions is of major importance in maintaining high productivity and selectivity. State of the art hydrocracking catalysts are generally dominated by an acid component. Acid catalyzed chemistry is initiated by a metal function in such catalysts by generating olefinic intermediates from paraffinic or cycloparaffinic precursors. There is general concensus in the literature that Group VIII metals are essentially equivalent for this purpose. The dominance of the acid function can lead to excessive cracking since primary products arising from ring opening are highly susceptible to acid cracking routes to lower molecular weight products. Thus, the primary focus on improving the performance of these bifunctional hydrocracking catalysts has been on optimizing the acid function. For these reasons, it remains difficult to control excessive cracking even in state of the art catalysts.

To maintain molecular weight of the products and to reduce volume of lower molecular weight fractions, the cleaving of side chains from ring compounds by the acid component needs to be minimized. Recent attempts to control acidity in zeolites include lowering of acidity with cation titration of residual acid sites. However, the approach to low acidity catalysts via dealumination of Y-type reaches a point of diminishing returns because excessive processing, such as multiple steam treatments, exchanges and Al extractions, which are needed to achieve the desired materials, results in major yield and crystallinity losses. Thus cation exchange is an alternate and simpler way to control acidity (though not as efficient or stable as control of Si/Al). Methods for controlling acidity have included ammonia titration in the process stream to exchange proton sites (PCT WO/92/13045). base exchange with alkali and alkali earth cations (Euro. Pat. Appl 0,519 573 Al) and re-alumination exchange methods developed by Lutz (Cryst. Res. Technol., 25, 921-6, (1990)) and others (PCT WO/93/25477).

Therefore, there is still a need in the art for a more selective ring opening process which can meet the objectives of: (i) reducing the number of ring structures in a product stream; (ii) avoiding significant dealkylation of any pendant substituents on the ring which reduces the volume of product in a specified boiling range: and (iii) and increasing volume swell by lowering the density of the product stream.

## Summary of the Invention

In contrast to the prior art. we have found that significant enhancements in ring opening selectivity are made by focussing on the metal function of the bifunctional catalyst. Contrary to the teachings of the prior art. we have found that all Group VIII metals are not equivalent for ring opening activity and selectivity. By focusing on the hydrogenolysis activity of these Group VIII metals, we have discovered that Ir has exceptional activity and selectivity for the target reaction of selective ring opening. Further, we have found that when dealing with multi-substituted $C_5$ ring compounds, additional enhancements in performance can be obtained by coupling this dominant hydrogenolysis activity with an effective, subordinate amount of controlled acidity, presumably via reducing the number of tertiary ring carbons.

In accordance with the present invention, there is provided a process for selectively opening rings of organic ring compounds present in a liquid, wherein

(a) at least some of the ring compounds contain at least one $C_5$ ring having at least one substituent whereby the average total number of carbon atoms thereof is at least 8 carbon atoms;

(b) the compounds are contacted with a catalyst compound in the presence of hydrogen at a temperature from 150°C to 400°C and a total pressure of 0.10 to 20.8 MPa (0 to 3,000 psig); and

(c) the catalyst comprises Ir on an alumina support, the Ir being present in amount up to 10 wt.% based on the total weight of the catalyst, which catalyst, when contacted with a feed comprised of 20 wt.% n-pentylcyclopentane in heptane diluent, at a temperature of 150°C to 350 °C, a hydrogen treat rate of 356 std $m^3/m^3$ (2,000 standard cubic feet per barrel) on liquid feed, a total pressure of 3.55 Mpa (500 psig), and a liquid hourly space velocity of at least 5 on liquid feed, will result in: a) at least a 25% yield of $C_{10}$ paraffins, and b) a selectivity to $C_{10}$ paraffin products of at least 0.80, which selectivity is defined by %$C_{10}$ paraffin yield/%$C_{10}$ ring disappearance.

In a preferred embodiment of the present invention, the process conditions in step (b) are 225° to 350°C and a total pressure from 0.79 to 10.44 MPa (100 to 1500 psig).

In another preferred embodiment of the present invention, the catalyst contains an effective amount of one or more performance enhancing transition metals, preferably metals selected from the 4th, 5th, and 6th periods of the Periodic Table of the Elements.

## Detailed Description of the Invention

The present invention is practiced on feedstreams containing ring compounds wherein at least a portion, preferably at least about 50% of the ring compounds contain at least one $C_5$ ring having at least one substituent therefrom, wherein the average number of carbon atoms for the $C_5$ ring compound is at least 8. Preferred feedstreams containing such compounds are those boiling in the distillate range (175°C to 400°C). Non-limiting examples of such feedstocks include diesel fuels, jet fuels, and heating oils. Preferrably these feedstocks have been hydrotreated to reduce sulfur content to low levels, preferrably less than 100 ppm, more preferrably below 10 ppm. Other feedstreams can also be treated in accordance with the present invention by the manipulation of catalyst and process conditions. Such other feedstreams include chemical feedstocks, such as those containing primarily benzenes, toluenes, and xylenes; as well as lube streams.

The instant process will impact the fuel characteristics of these feedstocks by reducing number of ring structures in the product stream, avoiding significant dealkylation of any pendant substituents on the ring which reduces the volume of product in a specified boiling range and increasing volume swell by lowering the density of the product stream. It is also desirable to produce distillate fuels with cetane numbers in excess of about 40, preferably in excess of about 45, and more preferably in excess of about 50. The cetane number of molecules within a class (e.g. normal paraffins) increases with the number of carbon atoms in the molecule. Molecular classes may be ranked in terms of their cetane number for a specific carbon number: normal paraffins have the highest cetane number followed by normal olefins, isoparaffins, and by monocyclic naphthenes. Aromatic molecules, particularly multi-ring aromatics, have the lowest cetane numbers.

For example, naphthalene has a cetane blending number of about 5-10; tetrahydronaphthalene (tetralin) about 15, decahydronaphthalene (decalin) about 35-38, butylcyclohexane about 58-62, and decane about 72-76. These cetane measurements are consistent with the trend for higher cetane value with increasing ring saturation and ring opening.

Further, the aromatics content of a distillate stream will vary depending on its source. For example, if the distillate stream is a product fraction from a crude distillation tower, then the stream will be relatively low in aromatics, particularly multi-ring aromatics, and have relatively high cetane numbers. Distillate streams which are product fractions from a fluid catalytic cracker, on the other hand, have relatively high amounts of aromatics, particularly multi-ring aromatics, and consequently have relatively low cetane numbers. It is known, by those having ordinary skill in the art that an increase in cetane number and cetane index may correspond to an increase in API gravity. Consequently, it is highly desirable to reduce the number of rings by selective ring opening.

Three terms commonly used in the literature to describe the transformation of naphthenes to paraffins or to naphthenes containing fewer rings, are "hydrogenolysis", "hydrodecyclization", and "ring opening". Hydrogenolysis reactions are those in which there is cleavage of a carbon-carbon bond, with addition of hydrogen at each point of cleavage. Hydrodecyclization is more specific in that a cyclic structure is cleaved in a hydrogen environment. Such reactions occur in the hydrocracking of large organic molecules, with formation of fragments that react with hydrogen in the presence of a suitable catalyst and at relatively high temperatures. Such fragments are typically either molecules in which rings have been cleaved, or are alkyl substituents which have been cleaved, or both. This results in products which contain fewer carbon atoms than the original molecule. This of course results in lower boiling products. "Ring opening" can simply be another way to describe hydrodecyclization. However, for purposes of the present invention, selective ring opening means a high propensity for cleavage of a ring bond which results in product molecules having substantially the same number of carbon atoms and one less ring than the original molecule.

The literature regarding the above terms are typically based on two types of experimental data - real feed data and model compound data. Examples of feeds on which data is reported in the literature include hydrogenated streams containing cyclic structures, such as hydrocracked products, aromatic hydrogenation products, and deasphalted oils. Streams which contain predominantly aromatics need to be hydrogenated first. Experimental data cited in the art for real feeds usually refer to the disappearance of rings in the products of interest for a particular process, or for total liquid product recovered. Because of the lack of appropriate analytical techniques and characterization tools, the reaction pathways and mechanisms leading to the disappearance of rings cannot be clearly identified and quantified. However, it is common in such reactions that there has been a substantial reduction in boiling point and/or average molecular weight of the product. Boiling point reduction and molecular weight reduction are evidence of non-selective ring opening. That is, characteristics of alkyl substituents to the ring being cleaved or cleavage of ring-opened products, or both. There is a substantial amount of literature on ring opening of model compounds, but it is typically limited to simple ring compounds having an alkyl group of only one or two carbon atoms. For example, the majority of experimental data is based on the conversion of methylcyclopentane, cyclohexane, and methylcyclohexane. Only a relatively small amount of data are based on the conversion of compounds having longer carbon substituent groups. such as butylcyclohexane, dimethyl- and trimethylcyclopentane.

Hydrogenolysis, as described in the present invention, is a key pathway for ring opening. Hydrogenolysis of naphthenes can be essentially described by the following two reactions: (1) the breaking of endocyclic carbon-carbon bonds; and (2) the breaking of exocyclic carbon-carbon bonds. The breaking of an endocyclic bond, as in ring opening, leads to a paraffin of the same carbon number weight for a one ring naphthene, or an alkylated naphthene of the same number of carbon atoms containing one less ring for a multiring naphthene. The breaking of an exocyclic carbon-carbon bond, as in dealkylation, results in the loss of an alkyl substituent which produces a decrease of molecular weight by producing two molecules of much lower boiling points.

Recognizing that the two reactions may occur co-currently or consecutively, it becomes necessary to define the concept of selective and non-selective ring opening and dealkylation. That is, selective ring opening without substantial dealkylation of alkyl substituents on the ring, and non-selective ring opening wherein ring opening is accompanied by substantial dealkylation of ring substituents. For that reason, it is necessary for chosing a selective ring opening catalyst to use a model compound such as butylcyclohexane which contains both a ring and a substituent containing a significant number of exocyclic carbon atoms. It is difficult to determine if a catalyst is selective for opening the ring as opposed to severing the alkyl substituent for cyclic compounds containing a substituent without a significant number of exocyclic carbon atoms, for example, methylcyclohexane. On the other hand, it is relatively easy to determine whether a catalyst is selectively opening the ring and not severing the substituent on compounds such as butylcyclohexane, which contain a ring substituent having 3 or more carbon atoms.

While preferred catalysts of the present invention will directly open five-membered naphthenic rings, it is possible that a catalyst system used herein for a feedstock containing multi-substituted rings may contain a controlled acid function which is effective for isomerizing substituents to change the site of attachment on the five-membered naphthenic rings. The ring opening function of the catalysts of the present invention will be more active when two adjacent ring carbons of the five-membered naphthenic rings are unsubstituted.

The instant process can be practised by contacting a suitable feedstream, containing the $C_5$ ring compounds, with the selective ring-opening Ir catalyst on an alumina support under process conditions of (a) temperatures from 150°C to 40°C, preferably from 225°C to 350°C; and (b) a total pressure from 0 to 20.8 MPa (3,000 psig), preferably from 0.79 to 1.62 MPa (100 to 2,200 psig); more preferably 0.79 to 10.44 Mpa (100 to 1,500 psig); a liquid hourly space velocity of 0.1 to 10, preferably from 0.5 to 5; and a hydrogen treat gas rate of 89 to 1780 std $m^3/m^3$ (500-10,000 standard cubic feet per barrel (SCF/B), preferably (1000-5000 SCF/B) 178 to 890 st $m^3/m^3$.

Preferably, in the Ir an alumina catalyst employed in the invention the amount of metal will be from 0.01 wt. % to 5 wt.%, more preferably from 0.02 wt.% to 3 wt.% and most preferably from 0.1 wt.% to 1 wt.%; all based on total wt of catalyst.

The selective ring opening catalysts used in the present invention can also include an effective amount of at least one performance enhancing transition meial, preferably those selected from the 4th, 5th, and 6th periods of the Periodic Table of the Elements. The Periodic Table of the Elements referred to herein is the type found on the last page of Advanced Inorganic Chemistry, by Cotton and Wilkinson. Interscience Publishers, 2nd Edition, 1966. Non-limiting examples of such metals include Cu, Zn, Ni, Pt, Re, Co, Mn, Mo, and W. Such performance enhancements include such things as sulfur protection, enhanced metals dispersion of the primary metal Ir and enhanced dehydroaenation activity.

If aromatics are present and need to be saturated, any suitable hydrotreating process can be used to hydrogenate the aromatic rings, so long as it is not associated with excessive cracking. Typical hydrotreating catalyst are comprised of at least one Group VIII metal and a Group VI metal on an inorganic refractory support, preferably alumina or an alumina-silica support. Said Groups are from the Periodic Table of the Elements. The Group VIII metal is present in an amount ranging from 2 to 20 wt.%, preferably from 4 to 12 w.%. Preferred Group VIII metals include Co, Ni, and Fe: with Co and Ni being more preferred. The preferred Group VI metal is Mo, which is present in an amount ranging from 5 to 50 wt.%, preferably from 10 to 40 wt.%, and more preferably from 20 to 30 wt.%. All metal weight percents are based on the total weight of the catalyst. Typical hydrotreating conditions for distillate feedstocks include temperatures from 200°C to 400°C, pressures from 0.79 to 10.44 MPa (100 to 1500 psig), space velocities from 0.4 to 6 V/V/Hr, and hydrogen gas rates from 200 to 6000 standard cubic feet per barrel (SCF/B).

The following examples are presented for illustrative purposes only and are not to be taken as limiting the present invention in any way.

## EXAMPLES

### Catalyst Selection Procedure

The following experimental procedure is used to select catalysts of the present invention which afford selective ring opening without excessive cleavage of substituent groups on the ring or product paraffins. The feedstock, prepared by dissolving 20 wt.% n-pentylcyclopentane (PCP), in heptane, contained 0.140 g, or 0.177 mL of PCP/mL at a density of 0.700 g/mL. The feedstock was filtered and delivered through a syringe pump to the test reactor run in a fixed-bed, downflow mode under essentially vapor phase conditions. Catalysts tested were about 14-35 mesh particle size. Product effluent passed from the reactor through a back pressure regulator, exiting at atmospheric pressure. The product effluent was analyzed by on-line gas chromotography and/or a combination of gas chromatography and mass spectrometry. The yield and conversion results (shown in the tables of results for the examples) were used to establish catalyst activity and selectivity parameters in the following manner.

• % PCP Conversion =

((g PCP in Feed - g PCP in Product) / g PCP in Feed) x 100 = % PCP

Conversion

• %$C_{10}$ Paraffin Yield (PY) =

(g $C_{10}$ Paraffins in Product / g PCP in Feed) x 100

• %$C_{10}$ Ring Disappearance (RD) =

(g PCP in Feed - (g PCP in Product + g $C_{10}$ Cycloparaffins in Product)) /

g PCP in Feed) x 100

• Selectivity for Ring opening (R-O) = (%PY/%RD)

% C10 Paraffin Yield / %$C_{10}$ Ring Disappearance = R-O Selectivity

**EXAMPLE 1** (0.9 wt.% Ir / $Al_2O_3$)

To illustrate this invention a catalyst was prepared in the following manner which is known in the art to give a well-dispersed metal catalyst (see U.S. Patent 4,302,359). The outlet of a large fritted-glass filtration funnel was equipped with a $CO_2$ gas delivery tube allowing the gas to flow upward through the glass frit. Into the funnel were placed 375 ml of de-ionized water and 250 g of reforming grade alumina extrudates. The $CO_2$ was bubbled through the mixture for 30 min. An Ir stock solution was prepared by the dissolution of 42.9 g of chloroiridic acid hexahydrate in 1l. of de-ionized water; the stock solution contained 16 mg Ir/ml and 18 mg Cl/ml. To the extrudate/water mixture was added 141 ml of Ir solution, and the passage of $CO_2$ was continued for 4 hr. The aqueous layer was decanted and the catalyst was dried overnight at room temperature on a bed of paper towels. The catalyst was subsequently dried under vacuum at 100°C for 4 hr. prior to being calcined in flowing air at 400°C for 3 hr. The resulting catalyst was 0.9 wt.% Ir on alumina.

A portion. 0.5 g (1.0 mL), of this 0.9 wt.% Ir/$Al_2O_3$ catalyst was tested for selective ring opening using the above described Catalyst Selection Procedure. The results. Table 1 below, show that the catalyst of Example 1 meets the preferred criteria for selective ring opening of the present invention.

**EXAMPLE 2** (0.9 wt.% Pt / $Al_2O_3$) - Comparative

A catalyst was prepared in a similar manner to that described in Example 1, except that chloroplatinic acid was used instead of chloroiridic acid, which resulted in a catalyst comprised of 0.9 wt.% Pt on alumina.

This catalyst, a 0.53 g (1.0 mL) portion, was tested for selective ring opening as described for Example 1. The results, by comparison with Example 1 in Table 1, show that 0.9 wt.% Pt / $Al_2O_3$ does not meet the minimum range of criteria for ring opening of the present invention, and thus is not a catalyst suitable for use this invention.

TABLE 1

| Comparison of Ring Opening Activity and Selectivity for Catalysts of Examples 1 and 2. | | | | | | | |
|---|---|---|---|---|---|---|---|
| Sample | Catalyst | Temp °C | Total, LHSV | PCP Conv | %$C_{10}$ RD | %$C_{10}$ PY | Selectivity |
| 1 | 0.9% Ir / $Al_2O_3$ | 250 | 5.0 | 65.0 | 65.0 | 59.8 | 0.92 |
| 2a | 0.9%Pt/$Al_2O_3$ | 300 | 5.0 | 5.0 | 4.3 | 3.7 | 0.86 |
| 2b | 0.9%Pt/$Al_2O_3$ | 300 | 0.5 | 91.0 | 81.0 | 61.9 | 0.76 |

These results of Examples 1 and 2a clearly show the difference in ring opening activity for iridium versus platinum, supporting the conclusion that all Group VIII metals are not equivalent for ring opening activity. The results of Example 2b reveal that in order to reach a paraffin yield similar to that for Example 1, an order of magnitude lower space velocity must be employed. However, at this level of conversion, the selectivity of the platinum catalyst falls below the minimum criteria for ring opening of this invention. Thus, the catalysts of Example 2 are not suitbale for use in this invention.

**EXAMPLE 3** 0.6 wt.% Ir - 0.1% Sn / $Al_2O_3$

A catalyst illustrating this invention was prepared in a manner similar to Example 1, except that the support was a commercial alumina prepared with a well dispersed tin content of 0.1 wt%. This catalyst, a 0.60 g (1.0 mL) portion, was tested for selective ring opening as described for Example 1. The results show that this iridium catalyst, which also contains tin, meets the minimum range of criteria for ring opening of the present invention, and thus is a catalyst suitable for use in this invention.

### EXAMPLE 4 0.6 wt.% Ir - 0.6% Pt / Al$_2$O$_3$

This catalyst, a 0.55 g (1.0 mL) portion, was tested for selective ring opening as described for Example 1 above and the results are shown in Table 2 below. The results show that this iridium catalyst, which also contains platinum, meets the minimum range of criteria for ring opening of the present invention, and thus is a catalyst suitbale for use in this invention.

TABLE 2

| Ring Opening Activity and Selectivity for the Catalysts of Examples 3 and 4 with Comparison to the Results from Example 1. | | | | | | | |
|---|---|---|---|---|---|---|---|
| Example | Catalyst | Temp °C | Tot. LHSV | PCP Conv. | %C$_{10}$ RD | %C$_{10}$ PY | Selectivity |
| 1 | 0.9% Ir / Al$_2$O$_3$ | 250 | 5.0 | 65.0 | 65.0 | 59.8 | 0.92 |
| 3 | 0.6%Ir-0.1%Sr Al$_2$O$_3$ | 250 | 5.0 | 43.0 | 43.0 | 37.8 | 0.88 |
| 4 | 0.6%Ir-0.6%Pr Al$_2$O$_3$ | 250 | 5.0 | 32.0 | 32.0 | 29.1 | 0.91 |

These results show that iridium ring opening catalysts may contain other transition elements from the fourth, fifth or sixth periods of the periodic table. The catalysts of both Examples 3 and 4 meet the criteria of this invention for ring opening.

### EXAMPLE 5 Ring opening of Tricyclo[5.2.1.0$^{2.6}$]decane over 0.9% Ilr/Al$_2$O$_3$.

A catalyst as described in Example 1, 0.93 g or 1.55 mL, was loaded into a tubular stainless steel reactor. The catalyst was prereduced in flowing hydrogen at 400 C for 1.5 hours at atmospheric pressure. A feed was prepared to contain 20 wt.% tricyclo[5.2.1.0$^{2.6}$]decane (TCD) in heptane diluent. The TCD is a tricyclic molecule of 10 carbon atoms containing 5 membered rings. This feed was passed over the catalyst at 500 psig. an LHSV of 13 and a hydrogen treat gas rate of 356 st m$^3$ / m$^3$ (2000 SCF/B). The results from this example are shown in Table 3.

TABLE 3

| Composition of Products from Ring Opening of Tricyclo [5.2.1.0$^{2,6}$]decane over 0.9 wt.% Ir / Al$_2$O$_3$ in Example 11 | | |
|---|---|---|
| Temp, °C | 275 | 290 |
| TCD Conv., % | 78.1 | 93.0 |
| C$_{10}$ 2-Ring Naphthenes. % | 47.3 | 49.5 |
| C$_{10}$ 1-Ring Naphthenes. % | 29.8 | 40.3 |
| C$_{10}$-Paraffins.% | 1.0 | 3:2 |

### EXAMPLE 6 Ring opening of Bicyclo[3.3.0]octane over 0.9% Ir / Al$_2$O$_3$

A catalyst as described in Example 1. 0.62 g or 1.10 mL, was loaded into a tubular stainless steel reactor. The catalyst was prereduced in flowing hydrogen at 400 C for 1.5 hours at atmospheric pressure. A feed was prepared to contain 20 wt% bicyclo[3.3,0]octane (BCO) in heptane diluent. The BCO is a bicyclic molecule of 8 carbon atoms containing two fused 5 membered rings. This feed was passed over the catalyst at 500 psig. an LHSV of 9.4 and a hydrogen treat gas rate of 2000 SCF/B. The results from this example are shown in Table 4 below.

### EXAMPLE 7 (Comparative) Ring opening of Bicyclo[3.3.0]octane over 0.9 wt.% Pt Al$_2$O$_3$

The catalyst described in Comparative Example 2, in amount 0.62 g or 1.10 mL, was loaded into a tubular stainless steel reactor and tested for ring opening as described in Example 6. The results from this example are shown in Table 4 below.

**EXAMPLE 8** (Comparative) Ring opening of Bicyclo[3.3.0]octane over 0.5 w-t.°% Ru / $Al_2O_3$

A commercial ruthenium on alumina catalyst, in amount 0.75 g or 1.00 mL, was loaded into a tubular stainless steel reactor and tested for ring opening as described in Example 6, except at LHSV 10.3. The results from this example are shown in Table 7 below.

TABLE 4

| Composition of Products from Ring Opening of Bicydo[3.3.0]octane over the Catalysts of Examples 6, 7 and 8 | | | | | | |
|---|---|---|---|---|---|---|
| Example | Catalyst | Temp °C | Tot. LHSV | BCO Conv. | %$C_8H_{16}$ 1-Ring Naphthenes | %$C_8H_{18}$ Paraffins |
| 6 | 0.9% Ir / $Al_2O_3$ | 225 | 9.4 | 100.0 | 17.8 | 81.4 |
| 7 | 0.9% Pt / $Al_2O_3$ | 300 | 9.4 | 13.8 | 13.8 | 0.0 |
| 8 | 0.5%Ru/$Al_2O_3$ | 200 | 10.3 | 69.9 | 48.9 | 19.6 |

These results illustrate, once again, the difference in ring opening activity of iridium versus platinum versus ruthenium, supporting the conclusion that not all Group VIII metals are equivalent for hydrogenolysis activity for ring opening.

**EXAMPLE 9** Ring opening of 2-Methylbicyclo[3.3.0]octane over 0.9 wt.% Ir / $Al_2O_3$

A catalyst as described in Example 1, 0.62 g or 1.10 mL, was loaded into a tubular stainless steel reactor. The catalyst was prereduced in flowing hydrogen at 400 C for 1.5 hours at atmospheric pressure. A feed was prepared to contain 20 wt% 2-methylbicyclo[3.3.0]octane (MeBCO) in heptane diluent. The MeBCO is a bicyclic molecule of 9 carbon atoms containing two fused 5 membered rings and a methyl substituent on one of the rings. This feed was passed over the catalyst at 500 psig, an LHSV of 9.4 and a hydrogen treat gas rate of 356 std $m^3/m^3$ (2000 SCF/B). The results from this example are shown in Table 5 below.

TABLE 5

| Composition of Products from Ring Opening of 2-Methylbicyclo[3.3.0]octane over the Catalyst of Example 9 | | | | | | |
|---|---|---|---|---|---|---|
| Example | Catalyst | Temp °C | Tot. LHSV | MeBCO Conv. | %$C_9H_{18}$ 1-Ring Naphthenes | %$C_9H_{20}$ Paraffins |
| 9 | 0.9% Ir / $Al_2O_3$ | 275 | 9.4 | 100.0 | 67.8 | 26.7 |

In this Example 9, the iridium catalyst gave very high ring opening selectivity, and further demonstrated ring opening of both five membered rings in the feed MeBCO resulting in a good yield, 26.7%, of $C_9$ paraffins, and with little cracking.

**EXAMPLE 10** Ring Opening activity and selectivity of 0.9% Ir / $Al_2O_3$ a Saturated Cyclic Feedstock

A catalyst described in Example 1, was tested for ring opening on a saturated cyclic feedstock prepared in the manner described for the above set of examples. The properties of this saturated cyclic feedstock. FS-16, are shown in Table 6 below. A 3.91 g (6.0 mL) portion 14-35 mesh particles of the catalyst of Example 1 was mixed with 4.0 mL inert diluent particles and loaded into a stainless steel reactor. The catalyst was prereduced in flowing hydrogen (150 mL/min) at 4.58 MPa (650 PSIG) under the following conditions: heat to 150°C at 38°C/hour; hold at 150°C for 13 hours: heat to 350°C at 100°C/hour: hold at 350°C for 17 hours. The saturated cyclic feedstock. FS-16. was passed over this catalyst mixture at the conditions shown in Table 7 below. The results in Table 7 show a decrease in number of cyclic 2-ring structures versus the relative boiling point conversion, which represents molecular weight reduction and cracking.

**EXAMPLE 11** (Comparative) Ring Opening activity and selectivity of 0.5 wt.% Pd / USY-Al$_2$O$_3$ (80-20) on a Saturated Cyclic Feedstock

A commercial hydrocracking catalyst comprised of 0.5 wt.% Pd supported on USY-Al$_2$O$_3$ (80-20) extrudates was tested for ring opening on a saturated cyclic feedstock prepared in the manner described in Examples 6 and 7 above. The properties of this saturated cyclic feedstock, FS-17, are shown in Table 6 below. A 0.63 g (1.0 mL) portion 14-35 mesh particles of the hydrocracking catalyst was mixed with 9.0 mL inert diluent particles and loaded into a stainless steel reactor. The catalyst was prereduced in flowing hydrogen (150 mL/min) at 4.55 MPa (650 PSIG) under the following conditions: heat to 150°C at 25°C/hour; hold at 150°C for 9.5 hours; heat to 350°C at 25°C/hour; hold at 300°C for 10 hours. The saturated cyclic feedstock, FS-17, was passed over this catalyst mixture at the conditions shown in Table 7 below. The results in Table 7 show a decrease in number of cyclic 2-ring structures versus the relative boiling point conversion, which represents molecular weight reduction and cracking.

TABLE 6

| Composition of Saturated Cyclic Feedstocks used in Examples 10 and 11 | | | |
|---|---|---|---|
| | | FS-10 Example 10 | FS-11 Example 11 |
| API Gravity | | 31.1 | 31.5 |
| Aromatics, by SFC | | 0.5 | 1.7 |
| Sulfur, ppm | | 0.06 | 0.1 |
| Nitrogen, ppm | | 0.1 | 0.9 |
| Boiling Range by GC Distillation | | | |
| % Boiling at °C (°F) | 0.5 | 181.6 (359) | 163.3 (326) |
| | 5 | 196.7 (386) | 191.1 (376) |
| | 10 | 203.3 (398) | 198.3 (389) |
| | 30 | 213.9 (417) | 206.7 (404) |
| | 50 | 220.0 (428) | 212.2 (414) |
| | 70 | 229.4 (445) | 222.2 (432) |
| | 90 | 247.8 (478) | 246.1 (475) |
| | 99.9 | 287.8 (550) | 291.1 (556) |
| % Paraffins | | 1 | 1 |
| % C$_{10}$+ 1 ring naphthenes | | 17 | 17 |
| % C$_{10}$+ 2-ring naphthenes | | 82 | 82 |

TABLE 7

| Comparison of Ring Opening Activity and Selectivity for Catalysts of Examples 10 and 11 (hydrogen treat gas rate held constant at 534 std m$^3$/m$^3$(3000 SCF/B)) | | | | | | |
|---|---|---|---|---|---|---|
| Example | Catalyst | Temp °C | Tot. LHSV | MPa (PSIG) | Total Liquid Product | |
| | | | | | Mole % 2-Rings | 375°F + Conv. |
| 10a | 0.9% Ir / Al$_2$O$_3$ | 325 | 0.5 | 4.58(650) | 55.8 | 13.4 |
| 10a | 0.5% Pd / USY-Al$_2$O$_3$ (80-20) | 275 | 5.0 | 4.58(650) | 57.6 | 37.8 |
| 10b | 0.9% Ir/Al$_2$O$_3$ | 350 | 0.5 | 4.58(650) | 33.1 | 29.0 |
| 11b | 0.5% Pd / USY-Al$_2$O$_3$ (80-20) | 314 | 5.0 | 4.58(650) | 37.1 | 59.5 |
| 10c | 0.9% Ir / Al$_2$O$_3$ | 350 | 0.5 | 7.0(1000) | 8.3 | 58.9 |

Comparing Examples 10 and 11 reveals that for similar conversion levels of 2-ring molecules in the feedstock, the level of boiling range conversion is significantly less for the iridium catalyst of Example 10 than for the hydrocracking catalyst of Example 11.

Comparing Examples 10b and 11b also reveal that for a higher, but also similar conversion levels of 2-ring molecules in the feedstock, the level of boiling range conversion is significantly less for the iridium catalyst of Example 16 than for the catalyst of Example 11.

Example 10c shows the high degree of 2-ring conversion possible over the iridium catalyst of Example 16 at a similar level of boiling range conversion to that for the catalyst of Example 11b.

Thus, the catalyst of Example 10 exhibits greater selectivity for 2-ring conversion versus boiling range conversion (overall reduction) than a hydrocracking-type catalyst.

**Claims**

1. A process for selectively opening rings of organic ring compounds present in a liquid, wherein

   (a) at least some of the ring compounds contain at least one $C_5$ ring having at least one substituent whereby the average total number of carbon atoms thereof is at least 8 carbon atoms;

   (b) the compounds are contacted with catalyst compound in the presence of hydrogen at a temperature from 150°C to 400°C and a total pressure of 0.10 to 20.8 MPa (0 to 3,000 psig); and

   (c) the catalyst comprises Ir on an alumina support, the Ir being present in amount up to 10 wt.% based on the total weight of the catalyst, which catalyst, when contacted with a feed comprised of 20 wt.% n-pentylcyclopentane in heptane diluent, at a temperature of 150°C to 350°C, a hydrogen treat rate of 356 std $m^3/m^3$ (2,000 standard cubic feet per barrel) on liquid feed, a total pressure of 3.55 Mpa (500 psig), and a liquid hourly space velocity of at least 5 on liquid feed, will result in at least a 25% yield of $C_{10}$ paraffins.

2. The process of claim 1, wherein the amount of metal is from 0.01 to 5 wt.%, preferably 0.1 to 1.0 wt.%.

3. The process of any preceding claim, wherein the catalyst, when reacted with the 20 wt.% n-pentylcyclopentane in heptane diluent, will result in at least a 50% yield of $C_{10}$ paraffins.

4. The process of any preceding claim, conducted in the presence of an effective amount of at least one performance-enhancing transition metal.

5. The process of claim 4, wherein the, or each transition metal is selected from the 4th, 5th, and 6th periods of the Periodic Table of the Elements.

6. The process of any preceding claim, wherein the said ring-compounds comprise alicyclic hydrocarbon compounds.

7. The process of any preceding claim, wherein the said ring compounds comprise a petroleum feedstock, for example a petroleum distillate.

**Patentansprüche**

1. verfahren zum selektiven Öffnen von Ringen organischer Ringverbindungen, die in einer Flüssigkeit vorhanden sind, bei dem

   (a) mindestens einige der Ringverbindungen mindestens einen $C_5$-Ring mit mindestens einem Substituenten enthalten, wodurch die durchschnittliche Gesamtzahl von dessen Kohlenstoffatomen mindestens 8 Kohlenstoffatome beträgt;
   (b) die Verbindungen mit einer Katalysatorverbindung in Gegenwart von Wasserstoff bei einer Temperatur von 150°C bis 4 und einem Gesamtdruck von 0,10 bis 20,8 MPa (0 bis 3 000 psig) in Kontakt gebracht werden; und
   (c) der Katalysator Ir auf einem Aluminiumoxidträger enthält, wobei das Ir in einer Menge von bis zu 10 Gew. %, bezogen auf das Gesamtgewicht des Katalysators, vorhanden ist, und wobei der Katalysator bei Kontakt mit einem Einsatzmaterial, das aus 20 Gew.% n-Pentylcyclopentan in Heptan als Verdünnungsmittel zusammengesetzt ist, bei einer Temperatur von 150°C bis 350°C, einer Wasserstoffbehandlungsrate von 356 Std-m³/m³ (2 000 Standard-Kubikfuß pro Barrel), bezogen auf flüssigem Einsatzmaterial, einem Gesamtdruck von 3,55 MPa (500 psig) und einem stündlichen Flüssigkeitsdurchsatz von mindestens 5, bezogen auf flüssigem Einsatzmaterial, zu mindestens einer 25 % Ausbeute an $C_{10}$-Paraffinen führt.

**2.** Verfahren nach Anspruch 1, bei dem die Menge an Metall 0,01 bis 5 Gew.%, vorzugsweise 0,1 bis 1,0 Gew.% beträgt.

**3.** Verfahren nach einem der vorhergehenden Ansprüche, bei dem der Katalysator bei Umsetzung mit den 20 Gew. % n-Pentylcyclopentan in Heptan als Verdünnungsmittel zu mindestens einer 50 % Ausbeute an $C_{10}$-Paraffinen führt.

**4.** Verfahren nach einem der vorhergehenden Ansprüche, das in Gegenwart einer wirksamen Menge von mindestens einem leistungssteigernden Übergangsmetall durchgeführt wird.

**5.** Verfahren nach Anspruch 4, bei dem das oder jedes Übergangsmetall ausgewählt ist aus der vierten, fünften und sechsten Periode des Periodensystems der Elemente.

**6.** Verfahren nach einem der vorhergehenden Ansprüche, bei dem die Ringverbindungen alicyclische Kohlenwasserstoffverbindungen enthalten.

**7.** Verfahren nach einem der vorhergehenden Ansprüche, bei dem die Ringverbindungen ein Petroleinsatzmaterial enthalten, beispielsweise ein Petroldestillat.

**Revendications**

**1.** Procédé d'ouverture sélective de cycles de composés cycliques organiques présents dans un liquide, dans lequel :

a) au moins certains des composés cycliques contiennent au moins un cycle en $C_5$ ayant au moins un substituant de sorte que le nombre total moyen d'atomes de carbone de celui-ci soit d'au moins 8 atomes de carbone;
b) les composés sont mis en contact avec un composé catalytique en présence d'hydrogène à une température de 150°C à 400°C et une pression manométrique totale de 0,10 à 20,8 MPa (0 à 3000 psig); et
c) le catalyseur comprend du Ir sur un support d'alumine, Ir étant présent en quantité jusqu'à 10% en poids par rapport au poids total du catalyseur, ledit catalyseur, lorsqu'il est mis en contact avec une charge comprenant 20% en poids de n-pentylcyclopentane dans un diluant d'heptane, à une température de 150°C à 350°C, une vitesse de traitement à l'hydrogène de 356 m³ std/m³ (2000 pieds cubes standard par baril) sur la charge liquide, une pression manométrique totale de 3,55 MPa (500 psig) et une vitesse spatiale horaire de liquide d'au moins 5 sur la charge liquide, donnant au moins un rendement de 25% de paraffines en $C_{10}$.

**2.** Procédé selon la revendication 1, dans lequel la quantité de métal est de 0,01 à 5% en poids, de préférence de 0,1 à 1,0% en poids.

**3.** Procédé selon l'une quelconque des revendications précédentes, dans lequel le catalyseur, lorsqu'il est soumis à une réaction avec les 20% en poids de n-pentylcyclopentane dans un diluant d'heptane, donnera au moins un rendement de 50% de paraffines en $C_{10}$.

**4.** Procédé selon l'une quelconque des revendications précédentes, réalisé en présence d'une quantité efficace d'au moins un métal de transition améliorant les performances.

**5.** Procédé selon la revendication 4, dans lequel le ou chaque métal de transition est choisi parmi la 4ème, la 5ème et la 6ème périodes du tableau périodique des éléments.

**6.** Procédé selon l'une quelconque des revendications précédentes, dans lequel lesdits composés cycliques comprennent des composés hydrocarbonés alicycliques.

**7.** Procédé selon l'une quelconque des revendications précédentes, dans lequel lesdits composés cycliques comprennent une charge pétrolière, par exemple un distillat de pétrole.